(19) Europäisches Patentamt
European Patent Office
Office européen des brevets

(11) Veröffentlichungsnummer: **0 259 738 B1**

(12) **EUROPÄISCHE PATENTSCHRIFT**

(45) Veröffentlichungstag der Patentschrift: **03.04.91**

(51) Int. Cl.5: **C07D 213/76**, C07D 401/06, C07D 413/06, C07D 417/06, A01N 43/00

(21) Anmeldenummer: **87112653.8**

(22) Anmeldetag: **31.08.87**

Die Akte enthält technische Angaben, die nach dem Eingang der Anmeldung eingereicht wurden und die nicht in dieser Patentschrift enthalten sind.

# (54) Heterocyclische Verbindungen.

(30) Priorität: **10.09.86 JP 211753/86**

(43) Veröffentlichungstag der Anmeldung:
**16.03.88 Patentblatt 88/11**

(45) Bekanntmachung des Hinweises auf die Patenterteilung:
**03.04.91 Patentblatt 91/14**

(84) Benannte Vertragsstaaten:
**AT BE CH DE ES FR GB GR IT LI NL SE**

(56) Entgegenhaltungen:
**EP-A- 0 136 636**
**EP-A- 0 154 178**
**EP-A- 0 163 855**
**EP-A- 0 192 060**
**EP-A- 0 199 974**
**EP-A- 0 212 600**
**US-A- 3 922 242**

**PESTIC. VENOM. NEUROTOX. 1976, Seiten 153-158; S.B. SOLOWAY et al.: "Nitromethylene heterocycles as insecticides"**

**CHEMICAL ABSTRACTS, Band 89, Nr. 13, 25 September 1978, Abs. 101812j**

(73) Patentinhaber: **NIHON TOKUSHU NOYAKU SEIZO K.K.**
**Itohpia Nihonbashi Honcho Building 7-1, Nihonbashi Honcho 2-chome**
**Chuo-ku Tokyo 103(JP)**

(72) Erfinder: **Shiokawa, Kozo**
**210-6, Shukugawara Tama-ku**
**Kawasaki-shi Kanagawa-ken(JP)**
Erfinder: **Tsuboi, Shinichi**
**3-26-1, Hirayama**
**Hino-shi Tokyo(JP)**
Erfinder: **Sasaki, Shoko**
**1-7-3, Higashi-Hirayama**
**Hino-shi Tokyo(JP)**
Erfinder: **Moriya, Koichi**
**5-7-11, Ueno Taito-ku**
**Tokyo(JP)**
Erfinder: **Hattori, Yumi**
**598, Kobiki-cho**
**Hachioji-shi Tokyo(JP)**
Erfinder: **Shibuya, Katsuhiko**
**39-15, Namiki-cho**
**Hachioji-shi Tokyo(JP)**

**EP 0 259 738 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

(74) Vertreter: **Schumacher, Günter, Dr. et al**
**c/o Bayer AG Konzernverwaltung RP Patentabteilung**
**W-5090 Leverkusen 1 Bayerwerk(DE)**

**Beschreibung**

Die vorliegende Erfindung betrifft neue heterocyclische Verbindungen, Verfahren zu ihrer Herstellung und ihre Verwendung als Insektizide.

Es ist bereits bekannt, daß bestimmte Pyridin-Resonanzhybride insektizide Wirksamkeit aufweisen (siehe die US-PS 3 922 242) und daß bestimmte 1-substituierte 1,2-Dihydro-2-nitroiminopyridine entzündungshemmende Wirkung besitzen (siehe J.Med.Chem. 1971, Band 14, Nr. 10, Seiten 988-990).

Aus der Publikation EP-A 0 192 060 sind ähnlich strukturierte Nitroiminoverbindungen bekannt.

Weitere Publikationen (US-A-3 922 242, EP-A 0 163 855, EP-A 0 154 178 und Adv.Pestic.Chem. 4th Int.Congr.Pest.Chem. Vol.2, 206-217(1979) beziehen sich ausschließlich auf Nitromethylenverbindungen, liegen also strukturell von den erfindungsgemäßen Verbindungen weiter entfernt als die Wirkstoffe gemäß EP-A 0 192 060.

Es wurden nun neue heterocyclische Verbindungen der Formel (I)

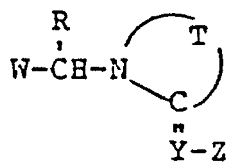

(I)

gefunden, in der

W eine Pyridyl-Gruppe mit wenigstens einem Substituenten ausgewählt aus Halogen-Atomen, $C_1$-$C_4$-Alkyl-Gruppen, die gegebenenfalls durch Halogen substituiert sind, $C_1$-$C_4$-Alkoxy-Gruppen, die gegebenenfalls durch halogen substituiert sind, $C_2$-$C_4$-Alkenyl-Gruppen, die gegebenenfalls durch Halogen substituiert sind, $C_1$-$C_4$-Alkylsulfinyl-Gruppen, $C_1$-$C_4$-Alkylsulfonyl-Gruppen und $C_3$-$C_4$-Alkinyl-Gruppen oder eine heterocyclische Gruppe, die zwei Hetero-Atome ausgewählt aus Sauerstoff-, Schwefel-und Stickstoff-Atomen enthält, wobei wenigstens eines der Hetero-Atome ein Stickstoff-Atom ist, und die gegebenenfalls durch einen Substituenten ausgewählt aus Halogen-Atomen, $C_1$-$C_4$-Alkyl-Gruppen, die gegebenenfalls durch Halogen substituiert sind, $C_1$-$C_4$-Alkoxy-Gruppen, die gegebenenfalls durch Halogen substituiert sind, $C_2$-$C_4$-Alkenyl-Gruppen, die gegebenenfalls durch Halogen substituiert sind, $C_1$-$C_4$-Alkylsulfinyl-Gruppen, $C_1$-$C_4$-Alkylsulfonyl-Gruppen und $C_3$-$C_4$-Alkinyl-Gruppen substituiert sein kann, ist
R ein Wasserstoff-Atom oder eine Methyl-Gruppe bezeichnet,
Y =N- bezeichnet,
Z eine Nitro-Gruppe oder eine Cyano-Gruppe bezeichnet und
T 3 oder 4 Ring-Glieder eines 5- oder 6-gliedrigen ungesättigten heterocyclischen Ringes bezeichnet, den diese zusammen mit dem benachbarten Kohlenstoff-Atom und dem benachbarten Stickstoff-Atom bilden, wobei der 5- oder 6-gliedrige ungesättigte heterocyclische Ring 1 bis 2 Hetero-Atome enthält, die aus Schwefel- und Stickstoff-Atomen ausgewählt sind und von denen wenigstens eines ein Stickstoff-Atom ist, wobei die 3 oder 4 Ring-Glieder gegebenenfalls durch wenigstens ein Halogen-Atom bzw. eine gegebenenfalls durch Halogen substituierte $C_1$-$C_4$-Alkyl-Gruppe substituiert sind.

Die Verbindungen der Formel (I) werden mit Hilfe eines Verfahren erhalten, bei dem

a) Verbindungen der Formel (II)

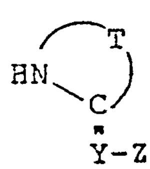

(II)

,

in der Y, Z und T die angegebenen Bedeutungen haben, mit Verbindungen der Formel (III)

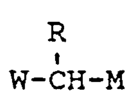

(III),

in der R und W die oben angegebenen Bedeutungen haben und M für ein Halogen-Atom oder die Gruppe-$OSO_2$-R' steht in der R' eine Alkyl- oder Aryl-Gruppe bezeichnet in Gegenwart eines inerten Lösungsmittels und gegebenenfalls in Gegenwart eienr Base umgesetzt werden.

Die neuen heterocyclischen Verbindungen der Formel (I) zeigen sehr stark ausgeprägte insektizide Eigenschaften.

Überraschenderweise zeigen die neuen heterocyclischen Verbindungen gemäß der Erfindung eine wesentlich größere insektizide Wirkung als Verbindungen, die aus dem oben angeführten Stand der Technik bekannt sind.

Von den erfindungsgemäßen Verbindungen der Formel (I) sind bevorzugte Verbindungen diejenigen, in denen

W eine Pyridyl-Gruppe mit einem Substituenten ausgewählt aus Fluoro, Chloro, Bromo, Methyl, Ethyl, Trifluoromethyl, Methoxy, Trifluoromethoxy, Vinyl, Allyl, Methylsulfinyl, Methylsulfonyl und Propargyl oder

eine ein Sauerstoff- oder Schwefel-Atom und ein Stickstoff-Atom enthaltende, 5-gliedrige heterocyclische Gruppe, die gegebenenfalls substituiert ist durch einen Substituenten ausgewählt aus Fluoro, Chloro, Bromo, Meyhyl, Ethyl, Trifluoromethyl, Methoxy, Trifluoromethoxy, Vinyl, Allyl, Methylsulfinyl, Methylsulfonyl und Propargyl, ist,

R ein Wasserstoff-Atom oder eine Methyl-Gruppe bezeichnet,

Y für =N- steht,

Z eine Nitro-Gruppe oder eine Cyano-Gruppe bezeichnet und

T 3 oder 4 Ring-Glieder eines Imidazolin-, Thiazolin-, Dihydropyridin- oder Dihydropyrimidin-Rings bezeichnen, den diese zusammen mit dem benachbarten Kohlenstoff-Atom und dem benachbarten Stickstoff-Atom bilden, wobei die Ring-Glieder gegebenenfalls durch Chloro oder Methyl substituiert sind.

Zu speziellen Beispielen für die Verbindungen der Formel (I) gemäß der Erfindung zählen

1-(2-Chloro-5-pyridylmethyl)-2-nitroimino-1,2-dihydro-pyridin,
1-(2-Chloro-5-pyridylmethyl)-5-methyl-2-nitroimino-1,2-dihydro-pyridin,
1-(3-Methyl-5-isoxazolylmethyl)-2-nitroimino-1,2-dihydro-pyridin,
3-(2-Chloro-5-pyridylmethyl)-2-nitroimino-4-thiazolin und
1-(2-Chloro-5-pyridylmethyl)-2-cyanoimino-1,2-dihydropyridin.

Wenn 2-Nitroimino-1,2-dihydropyridin und 2-Chloro-5-chloromethylpyridin als Ausgangsstoffe in dem Verfahren a) eingesetzt werden, läßt sich die Reaktion durch das nachstehende Schema veranschaulichen.

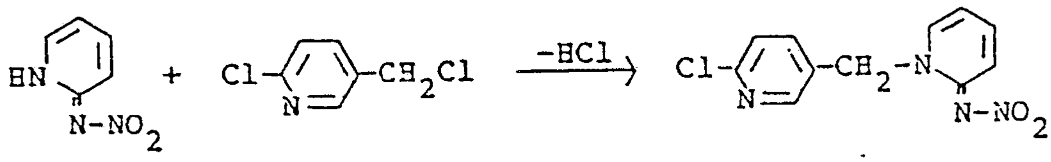


Im Verfahren a) bezeichnet die Verbindung der Formel (II) eine auf den vorstehenden Definitionen von Y, Z und T basierende Verbindung.

In der Formel (II) sind Y, Z und T vorzugsweise synonym mit den im Vorstehenden angegebenen bevorzugten Definitionen.

Die Formel (II) umfaßt sowohl bekannte als auch neue Verbindungen.

Die Verbindungen der Formel (II) können in Resonanz-Struktur existieren, wie nachstehend dargestellt ist.

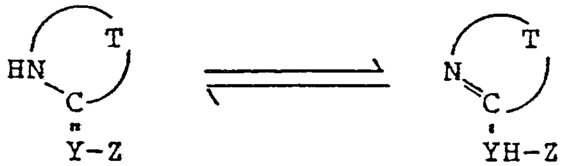

(II) (II')

Im Hinblick auf die obige Resonanz-Struktur zählen zu Beispielen für die bekannten Verbindungen der Formel (II)

2-Nitromethylpyridin und seine in J. Am. Chem. Soc., Band 91 , Seiten 1856-1857, beschriebenen kernalkyl-substituierten Produkte:
4-Nitromethylpyrimidine,beschrieben in J. Org. Chem., Band 37 , Seiten 3662-3670;
3-Nitromethyl-1,2,5-oxadiazol, beschrieben in Liebigs Ann. Chem., 1975, Seiten 1029 bis 1050;
2-Pyridylacetonitril, beschrieben in J. Am. Chem. Soc., Band 73 , Seiten 5752-5759;
5-Imidazolylacetonitril, beschrieben in Chem. Abstr., Band 50 , 15516a;
2-Imidazolylacetonitril, beschrieben in J. Med. Chem., Band 11 , Seiten 1028-1031;
2-Pyrimidylacetonitril, 2-Thiazolylacetonitril und 4-Thiazolylacetonitril, beschrieben in der JP-OS 49972/1974;
2-Nitroiminopyridin, beschrieben in J. Med. Chem., Band 14 , Seiten 988-990;
5-Chloro-2-nitroiminopyridin, beschrieben in Beilstein, 22 II, Seite 519;
methyl-substituiertes 2-Nitroiminopyridin, beschrieben in J. Am. Chem. Soc., Band 77 , Seiten 3154-3155;
5-Chloro-2-nitroiminopyrimidin, beschrieben in der US-PS 3 041 339;
3-Methyl-6-nitroiminopyridazin und 3-Nitroiminopyridazin, beschrieben in J. Chem. Soc., 1950, Seiten 3236-3239;
3-Chloro-6-nitroiminopyridazin, beschrieben in Chem. Abstr., Band 55 , 1634i;
2-Nitroiminothiazol, beschrieben in Can. J. Chem., Band 31 , Seiten 885-893;
2-Nitroimino-4-trifluoromethylthiazol, beschrieben in J. Org. Chem., Band 20 , Seiten 499-510;
methyl-substituiertes 2-Nitroiminothiazol, beschrieben in Can. J. Chem., Band 34 , Seiten 1291-1270;
4-Nitroimino-1,2,3-thiadiazol, beschrieben in J. Chem. Soc., 1965, Seite 5175;
3-Methyl-5-nitroimino-1,2,4-thiadiazol, beschrieben in der BE-PS 619 423;
2-alkyl- oder -halogen-substituiertes 5-Nitroimino-1,3,4-thiadiazol, beschrieben in J. Pharm. Soc. Japan, Band 75 , Seiten 1149-1150, oder der JP-Patentveröffentlichung Nr. 9736/1977;
2-Cyanoiminopyridin, beschrieben in Ann. Pharm. Fr., Band 26 , Seiten 469-472;
2-Cyanomethylthiazol, beschrieben in Chem. Pharm. Bull., Band 21 , Seiten 74-86;
2-Cyanoiminopyrimidin, beschrieben in der GB-PS 860 423 und
2-Cyanomethylpyridin, beschrieben in Chem. Ber., Band 85 , Seiten 397-407.

Ethyl-2-nitro-2-(2-pyridyl)acetat kann in einfacher Weise durch Nitrieren von Ethyl-2-pyridylacetat nach der in J. Ogr. Chem., Band 37 , Seiten 3662-3670, beschriebenen Methode erhalten werden.

Im Verfahren a) bezeichnet die Verbindung der Formel (III) als der andere Ausgangsstoff eine auf den vorstehenden Definitionen von R, W und M basierende Verbindung.

In der Formel (III) sind R und W vorzugsweise synonym mit den im Vorstehenden angegebenen bevorzugten Definitionen, und M bezeichnet vorzugsweise Chloro, Bromo oder Tosyloxy.

Die Verbindungen der Formel (III) sind bekannt, und typische Beispiele dafür sind
2-Chloro-5-chloromethylpyridin,
5-Chloromethyl-3-methylisoxazol,
5-Chloromethyl-2-chlorothiazol,
5-Chloromethyl-2-methylthiazol,
5-Chloromethyl-2-fluoropyridin,
2-Bromo-5-chloromethylpyridin und
5-Chloromethyl-2-methylpyridin.

Bei der praktischen Durchführung des Verfahrens a) können alle inerten organischen Lösungsmittel als geeignete Verdünnungsmittel verwendet werden.

Zu Beispielen für solche Verdünnungsmittel zählen Wasser; aliphatische, alicyclische oder aromatische (gegebenenfalls chlorierte) Kohlenwasserstoffe wie Hexan, Cyclohexan, Petrolether, Ligroin, Benzol, Toluol, Xylol, Methylenchlorid, Chloroform, Kohlenstofftetrachlorid, Ethylenchlorid und Chlorbenzol; Ether wie Diethylether, Methylethylether, Di-isopropylether, Dibutylether, Propylenoxid, Dioxan und Tetrahydrofuran; Ketone wie Aceton, Methylethylketon, Methylisopropylketon und Methylisobutylketon; Nitrile wie Acetonitril, Propionitril und Acrylnitril; Alkohole wie Methanol, Ethanol, Isopropanol, Butanol und Ethylenglycol; Ester wie Ethylacetat und Amylacetat; Säureamide wie Dimethylformamid und Dimethylacetamid; Sulfone und Sulfoxide wie Dimethylsulfoxid und Sulfolan; und Basen wie Pyridin.

Beispiele für die einsetzbare Base sind anorganische Basen wie Natriumhydroxid und Kaliumcarbonat und organische Basen wie Triethylamin.

Das Verfahren a) kann in einem weiten Temperaturbereich praktisch durchgeführt werden, beispielsweise bei einer Temperatur von etwa 0 °C bis 120 °C, vorzugsweise von etwa 20 °C bis etwa 80 °C.

Vorzugsweise wird die Reaktion unter Atmosphärendruck durchgeführt, jedoch ist es auch möglich, bei

erhöhtem oder vermindertem Druck zu arbeiten.

Bei der praktischen Durchführung des Verfahrens a) kann die gewünschte Verbindung der Formel (I) dadurch erhalten werden, daß 1 mol der Verbindung der Formel (II) mit etwa 1 bis 1,2 mol Triethylamin, und etwa 1 bis 1,2 mol, vorzugsweise 1 mol, der Verbindung der Formel (III) in einem inerten Lösungsmittel wie Ethanol umgesetzt wird.

Die Verbindungen der Formel (I) der vorliegenden Erfindung können eine Resonanz-Struktur annehmen, wie nachstehend gezeigt wird.

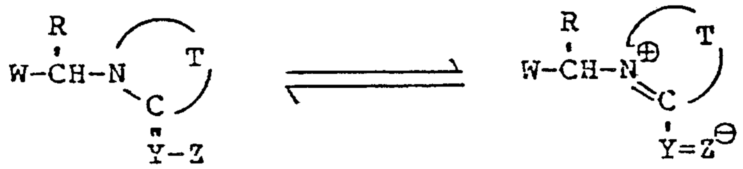

(I) (I')

Wenn das Ende von T auf der C-Seite ein Stickstoff-Atom ist, kann die Verbindung der Formel (I) auch die folgende Resonanz-Struktur annehmen.

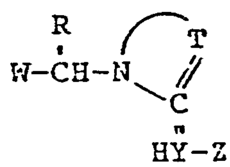

(II')

Vorzugsweise werden die Verbindungen der vorliegenden Erfindung durch die Formel (I) dargestellt.

Die aktiven Verbindungen werden von Pflanzen gut vertragen, haben einen günstigen Wert der Toxizität gegenüber warmblütigen Tieren und lassen sich einsetzen zur Bekämpfung von Arthropoden-Schädlingen, insbesondere von Insekten, die in der Land- und Forstwirtschaft auftreten, zum Schutz von Lagerprodukten und Materialien und auf dem Gebiet der Hygiene. Sie sind gegenüber normal empfindlichen und resistenten Species sowie gegen alle oder einige Entwicklungsstadien aktiv. Zu den oben genannten Schädlingen zählen:

Aus der Klasse der Isopoda beispielsweise
- Oniscus asellus,
- Armadillidium vulgare und
- Porcellio scaber;

aus der Klasse der Diplopoda beispielsweise
- Blaniulus guttulatus;

aus der Klasse der Chilopoda beispielsweise
- Geophilus carpophagus und
- Scutigera spec.;

aus der Klasse der Symphyla beispielsweise
- Scutigerella immaculata;

aus der Ordnung der Thysanura beispielsweise
- Lepisma saccharina;

aus der Ordnung der Collembola beispielsweise
- Onychiurus armatus;

aus der Ordnung der Orthoptera beispielsweise
- Blatta orientalis,
- Periplaneta americana,
- Leucophaea maderae,
- Blatella germanica,
- Acheta domesticus,
- Gryllotalpa spp.,
- Locusta migratoria migratorioides,
- Melanoplus differentialis und
- Schistocerca gregaria;

aus der Ordnung der Dermaptera beispielsweise
Forficula auricularia,
aus der Ordnung der Isoptera beispielsweise
Reticulitermes spp.;
aus der Ordnung der Anoplura beispielsweise
Phylloxera vastatrix,
Pemphigus spp.,
Pediculus humanus corporis,
Haematopinus spp. und
Linognathus spp.;
aus der Ordnung der Mallophaga beispielsweise
Trichodectes spp. und
Damalinea spp.;
aus der Ordnung der Thysanoptera beispielsweise
Hercinothrips femoralis und
Thrips tabaci;
aus der Ordnung der Heteroptera beispielsweise
Eurygaster spp.,
Dysdercus intermedius,
Piesma quadrata,
Cimex lectularius,
Rhodnius prolixus und
Triatoma spp.;
aus der Ordnung der Homoptera beispielsweise
Aleurodes brassicae,
Bemisia tabaci,
Trialeurodes vaporariorum,
Aphis gossypii,
Brevicoryne brassicae,
Cryptomyzus ribis,
Aphis fabae,
Doralis pomi,
Eriosoma lanigerum,
Hyalopterus arundinis,
Macrosiphum avenae,
Myzus spp.,
Phorodon humuli,
Rhopalosiphum padi,
Empoasca spp.,
Euscelis bilobatus,
Nephotettix cincticeps,
Lecanium corni,
Saissetia oleae,
Laodelphax striatellus,
Nilaparvata lugens,
Aonidiella aurantii,
Aspidiotus hederae,
Pseudococcus spp. und
Psylla spp.;
aus der Ordnung der Lepidoptera beispielsweise
Pectinophora gossypiella,
Bupalus piniarius,
Cheimatobia brumata,
Lithocolletis blancardella,
Hyponomeuta padella,
Plutella maculipennis,
Malacosoma neustria,
Euproctis chrysorrhoea,

Lymantria spp.,
Bucculatrix thurberiella,
Phyllocnistis citrella,
Agrotis spp.,
Euxoa spp.,
Feltia spp.,
Earias insulana,
Heliothis spp.,
Spodoptera exigua,
Mamestra brassicae,
Panolis flammea,
Prodenia litura,
Spodoptera spp.,
Trichoplusia ni,
Carpocapsa pomonella,
Pieris spp.,
Chilo spp.,
Pyrausta nubilalis,
Ephestia kuehniella,
Galleria mellonella,
Cacoecia podana,
Capua reticulana,
Choristoneura fumiferana,
Clysia ambiguella,
Homona magnanima und
Tortrix viridana;

aus der Ordnung der Coleoptera beispielsweise
Anobium punctatum,
Rhizopertha dominica,
Acanthoscelides obtectus,
Hylotrupes bajulus,
Angelastica alni,
Leptinotarsa decemlineata,
Phaedon cochleariae,
Diabrotica spp.,
Psylliodes chrysocephala,
Epilachna varivestis,
Atomaria spp.,
Oryzaephilus surinamensis,
Anthonomus spp.,
Sitophilus spp/.
Otiorrhynchus sulcatus,
Cosmopolites sordidus,
Ceuthorrhynchus assimilis,
Hypera postica,
Dermestes spp.,
Trogoderma spp.,
Anthrenus spp.,
Attagenus spp.,
Lyctus spp.,
Meligethes aeneus,
Ptinus spp.,
Niptus hololeucus,
Gibbium psylloides,
Tribolium spp.,
Tenebrio molitor,
Agriotes spp.,
Conoderus spp.,

Melolontha melolontha,
Amphimallon solstitialis und
Costelytra zealandica;

aus der Ordnung der Hymenoptera beispielsweise
Diprion spp.,
Hoplocampa spp.,
Lasius spp.,
Monomorium pharaonis und
Vespa spp.;

aus der Ordnung der Diptera beispielsweise
Aedes spp.,
Anopheles spp.,
Culex spp.,
Drosophila melanogaster,
Musca spp.,
Fannia spp.,
Calliphora erythrocephala,
Lucilia spp.,
Chrysomyia spp.,
Cuterebra spp.,
Gastrophilus spp.,
Hyppobosca spp.,
Stomoxys spp.,
Oestrus spp.,
Hypoderma spp.,
Tabanus spp.,
Tannia spp.,
Bibio hortulanus,
Oscinella frit,
Phorbia spp.,
Pegomyia hyoscyami,
Ceratitis capitata,
Dacus oleae und
Tipula paludosa.

Auf dem Gebiet der Veterinärmedizin sind die neuen Verbindungen der vorliegenden Erfindung gegen verschiedene schädliche Tierparasiten (innere und äußere Parasiten) wie Insekten und Würmer wirksam.

Beispielen für solche Tierparasiten sind Insekten wie
Gastrophilus spp.,
Stomoxys spp.,
Trichodectes spp.
Rhodnius spp. und
Ctenocephalides canis.

Die Wirkstoffe können in gebräuchliche Formulierungen überführt werden, etwa Lösungen, Emulsionen, Suspensionen, Pulver, Schäume, Pasten, Granulat, Aerosol, mit dem Wirkstoff imprägnierte natürliche oder synthetische Stoffe, sehr feine Kapseln in polymeren Substanzen, Überzugsmassen zur Verwendung auf Saatgut (Beizmittel) sowie Formulierungen für den Einsatz mit Verbrennungseinrichtungen wie Räucherpatronen, Räucherdosen und Räucherschlangen sowie für die kalte Vernebelung und die warme Vernebelung nach dem Ultra-Low-Volume-Verfahren.

Diese Formulierungen können in bekannter Weise hergestellt werden, beispielsweise durch Vermischen der Wirkstoffe mit Streckmitteln, das heißt mit flüssigen oder verflüssigten gasförmigen oder festen Verdünnungsmitteln oder Trägern, gegebenenfalls unter Verwendung grenzflächenaktiver Mittel, das heißt von Emulgatoren und/oder Dispergiermitteln und/oder schaumbildenden Mitteln. Bei Verwendung von Wasser als Streckmittel können organische Lösungsmittel beispielsweise als Hilfslösungsmittel verwendet werden.

Als flüssige Lösungsmittel, Verdünnungsmittel oder Träger vorzugsweise geeignet sind aromatische Kohlenwasserstoffe wie Xylol, Toluol oder Alkylnaphthaline, chlorierte aromatische oder chlorierte aliphatische Kohlenwasserstoffe wie Chlorbenzole, Chloroethylene oder Methylenchlorid, aliphatische Kohlenwasserstoffe wie Cyclohexan oder Paraffine, beispielsweise Mineralöl-Fraktionen, Alkohole wie Butanol oder

Glycol sowie deren Ether und Ester, Ketone wie Aceton, Methylethylketon, Methylisobutylketon oder Cyclohexanon oder stark polare Lösungsmittel wie Dimethylformamid und Dimethylsulfoxid sowie auch Wasser.

Unter verflüssigten gasförmigen Verdünnungsmitteln oder Trägern sind Flüssigkeiten zu verstehen, die bei normaler Temperatur und normalem Druck gasförmig wären, beispielsweise Aerosol-Treibmittel wie halogenierte Kohlenwasserstoffe sowie Butan, Propan, Stickstoff und Kohlenstoffdioxid.

Als feste Träger verwendbar sind gemahlene natürliche Minerale wie Kaoline, Tone, Talkum, Kreide, Quarz, Attapulgit, Montmorillonit oder Diatomeenerde und gemahlene synthetische Minerale wie hochdisperse Kieselsäure, Aluminiumoxid und Silicate. Als feste Träger für Granulat können zerkleinerte und fraktionierte Natursteinmaterialien verwendet werden, etwa Calcit, Marmor, Bimsstein, Sepiolith und Dolomit sowie synthetisches Granulat aus anorganischen und organischen Mehlen und Granulat aus organischem Material wie Sägemehl, Kokosnußschalen, Maiskolben und Tabakstengel.

Als emulgierende und/oder schaumbildende Mittel können nicht-ionische und anionische Emulgatoren wie Polyoxyethylenfettsäureester, Polyoxyethylenfettalkoholether, beispielsweise Alkylarylpolyglycol-Ether, Alkylsulfonate, Alkylsulfate, Arylsulfonate sowie Albumin-Hydrolyseprodukte verwendet werden. Zu Dispergiermitteln zählen beispielsweise Ligninsulfit-Ablaugen und Methylcellulose.

Haftmittel wie Carboxymethylcellulose und natürliche und synthetische Polymere in Form von Pulvern, Granulat oder Latices wie Gummi arabicum, Polyvinylalkohol und Polyvinylacetat können bei der Formulierung verwendet werden.

Es ist möglich, farbgebende Mittel, etwa anorganische Pigmente wie beispielsweise Eisenoxid, Titanoxid und Preußisch Blau und organische Farbstoffe wie Alizarin-Farbstoffe, Azo-Farbstoffe oder Metallphthalocyanin-Farbstoffe, sowie Spuren-Nährstoffe wie Salze von Eisen, Mangan, Bor, Kupfer, Cobalt, Molybdän und Zink zu verwenden.

Die Formulierungen enthalten im allgemeinen 0,1 bis 95 Gew.-%, vorzugsweise 0,5 bis 90 Gew.-% des Wirkstoffs.

Die erfindungsgemäßen Wirkstoffe können in ihren handelsüblichen Formulierungen oder den aus diesen Formulierungen hergestellten Anwendungsformen im Gemisch mit anderen Wirkstoffen vorliegen, etwa mit Insektiziden, Ködern, Sterilisationsmitteln, Akariziden, Nematiziden, Fungiziden, Wachstumsregulatoren oder Herbiziden. Zu den Insektiziden gehören beispielsweise Phosphate, Carbamate, Carboxylate, chlorierte Kohlenwasserstoffe, Phenylharnstoffe und von Mikroorganismen erzeugte, biologisch aktive Substanzen.

Die erfindungsgemäßen Wirkstoffe können weiterhin in ihren handelsüblichen Formulierungen oder den aus diesen Formulierungen hergestellten Anwendungsformen im Gemisch mit synergistischen Mitteln vorliegen. Synergistische Mittel sind Verbindungen, die die Wirkung der Wirkstoffe steigern, ohne daß es für das zugesetzte synergistische Mittel erforderlich ist, selbst wirksam zu sein.

Der Gehalt des Wirkstoffs in den aus den im Handel erhältlichen Formulierungen hergestellten Anwendungsformen kann innerhalb weiter Grenzen variieren. Die Konzentration der aktiven Verbindung in den Anwendungsformen kann 0,0000001 bis 100 Gew.-% betragen und liegt vorzugsweise zwischen 0,0001 und 1 Gew.-%.

Die Verbindungen werden in üblicher Weise in einer den Anwendungsformen angemessenen Form zur Anwendung gebracht.

Bei der Verwendung gegen Hygiene-Schädlinge und Schädlinge in Produkt-Vorräten zeichnen sich die Wirkstoffe durch eine hervorragende Rückstandswirkung auf Holz und Ton sowie eine gute Beständigkeit gegen Alkali auf gekalkten Unterlagen aus.

Die folgenden Beispiele erläutern die vorliegende Erfindung im einzelnen. Es ist jedoch ausdrücklich darauf hinzuweisen, daß die vorliegende Erfindung in keiner Weise allein auf diese Beispiele beschränkt ist.

Herstellungsbeispiele

Beispiel 1

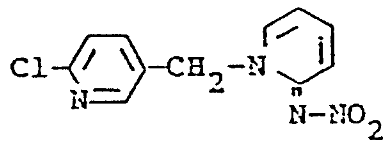

(Verbindung Nr. 1)

2-Chloro-5-chloromethylpyridin (3,24 g) und 2-Nitraminopyridin (2,78 g) wurden in wasserfreiem Ethanol (50 ml) gelöst, und Triethylamin (4,04 g) wurde zu der Lösung hinzugefügt. Die Mischung wurde eine Weile bei Raumtemperatur gerührt. Dann wurde sie 8 Stunden lang unter Rückfluß-Bedingungen erhitzt. Die Reaktionsmischung wurde auf Raumtemperatur abgekühlt und in Eiswasser gegossen. Die ausgefallenen Kristalle wurden durch Filtration gesammelt und aus Ethanol umkristallisiert, wonach blaßgelbes 1-(2-Chloro-5-pyridylmethyl)-2-nitroimino-1,2-dihydropyridin (2,3 g) als gewünschte Verbindung erhalten wurde; Schmp. 209-212 °C.

Beispiel 2

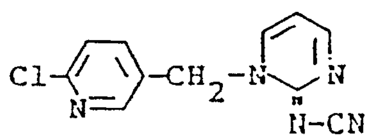

(Verbindung Nr. 25)

Eine Mischung von 2-Chloro-5-chloromethylpyridin (3,24 g), 2-Cyanaminopyrimidin (2,4 g), wasserfreiem Kaliumcarbonat (3,04 g) und Acetonitril (100 ml) wurde 5 Stunden lang unter Rühren zum Rückfluß erhitzt. Etwa 50 ml des Acetonitrils wurden unter vermindertem Druck abdestilliert, und der Rückstand wurde in Eiswasser gegossen. Die abgeschiedenen Kristalle wurden durch Filtration gesammelt und aus Ethanol umkristallisiert, wonach 1-(2-Chloro-5-pyridylmethyl)-2-cyanoimino-1,2-dihydropyrimidin (1,87 g) in Form farbloser Kristalle erhalten wurde; Schmp. 218-220 °C.

In der nachstehenden Tabelle 1 sind, zusammen mit den in den Beispielen 1 und 2 erhaltenen Verbindungen, Verbindungen der vorliegenden Erfindung aufgeführt, die nach den gleichen Methoden erhalten wurden, wie sie in den Beispielen 1 und 2 aufgeführt sind.

## Tabelle 1

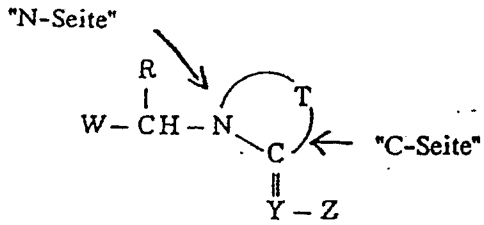


| Verbindung Nr. | W | R | N-Seite T C-Seite | Y | Z | |
|---|---|---|---|---|---|---|
| 1 | 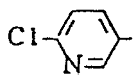 | H | $-CH=CH-CH=CH-$ | =N– | $NO_2$ | Schmp. 209~212 ℃ |
| 2 | 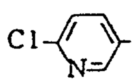 | H | $CH_3$<br>$-CH=C-CH=CH-$ | =N– | $NO_2$ | Schmp. 188~190 ℃ |
| 3 | 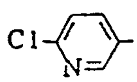 | H | $CH_3$<br>$-CH=CH-CH=C-$ | =N– | $NO_2$ | $n_D^{20}$ 1.5939 |
| 4 | 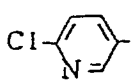 | H | Cl<br>$-CH=C-CH=CH-$ | =N– | $NO_2$ | Schmp. 84~85 ℃ |
| 5 | 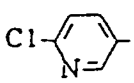 | H | Br<br>$-CH=C-CH=CH-$ | =N– | $NO_2$ | Schmp. 210~217 ℃ |
| 6 | 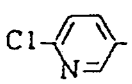 | H | Br Br<br>$-CH=C-CH=C-$ | =N– | $NO_2$ | Schmp. 229~232 ℃ |
| 7 | 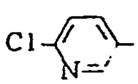 | H | $CF_3$<br>$-CH=C-CH=CH-$ | =N– | $NO_2$ | Schmp. 159~160 ℃ |
| 8 | 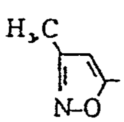 | H | $-CH=CH-CH=CH-$ | =N– | $NO_2$ | Schmp. 159~161 ℃ |
| 9 | 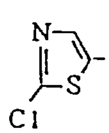 | H | $-CH=CH-CH=CH-$ | =N– | $NO_2$ | Schmp. 87~88 ℃ |
| 10 | 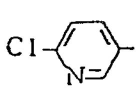 | H | $-CH=CH-S-$ | =N– | $NO_2$ | Schmp. 219~220 ℃ |
| 11 | 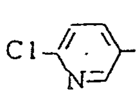 | H | $-N=CH-S-$ | =N– | $NO_2$ | Schmp. 166~167 ℃ |

| Verbindung Nr. | W | R | N-Seite T C-Seite | Y | Z | |
|---|---|---|---|---|---|---|
| 12 | 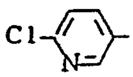 | H | 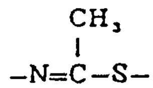 | =N– | $NO_2$ | Schmp.180~183 ℃ |
| 13 | 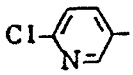 | H | 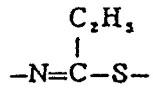 | =N– | $NO_2$ | Schmp.167~170 ℃ |
| 14 | 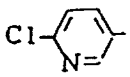 | H | 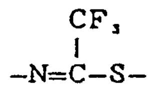 | =N– | $NO_2$ | Schmp.178~180 ℃ |
| 15 | 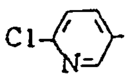 | H | 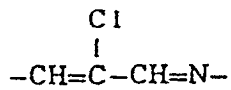 | =N– | $NO_2$ | Schmp.90~95 ℃ |
| 16 | 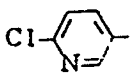 | H | 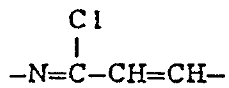 | =N– | $NO_2$ | $n_D^{20}$ 1.6335 |
| 17 | 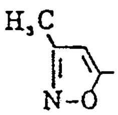 | H | –CH=CH–S– | =N– | $NO_2$ | Schmp.224~227 ℃ |
| 18 | 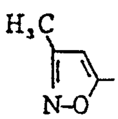 | H | 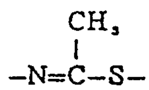 | =N– | $NO_2$ | Schmp.137~140 ℃ |
| 19 | 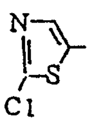 | H | 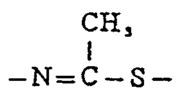 | =N– | $NO_2$ | Schmp.122~124 ℃ |
| 20 | 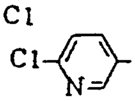 | H | –CH=CH–CH=CH– | =N– | CN | Schmp.184~186 ℃ |
| 21 | 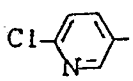 | H | 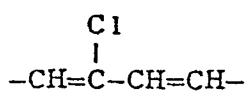 | =N– | CN | Schmp.215– 219 ℃ |
| 22 | 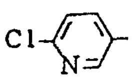 | H | 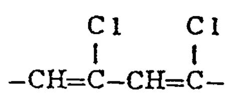 | =N– | CN | |
| 23 | 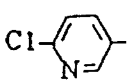 | H | –CH=CH–S– | =N– | CN | Schmp.151~154 ℃ |
| 24 | 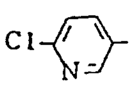 | H | –N=CH–S– | =N– | CN | Schmp.122~124 ℃ |
| 25 | 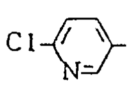 | H | –CH=CH–CH=N– | =N– | CN | Schmp.218~220 ℃ |

| Verbindung Nr. | W | R | N-Seite T C-Seite | Y | Z | |
|---|---|---|---|---|---|---|
| 26 | 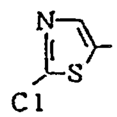 | H | −CH=CH−CH=CH− | =N− | CN | Schmp: 218~219 ℃ |
| 27 | 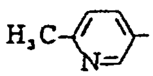 | H | −CH=CH−CH=CH− | =N− | $NO_2$ | |
| 28 | 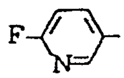 | H | −CH=CH−CH=CH− | =N− | $NO_2$ | |
| 29 | 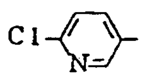 | $CH_3$ | −CH=CH−CH=CH− | =N− | $NO_2$ | |
| 30 | 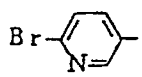 | H | −CH=CH−CH=CH− | =N− | $NO_2$ | |
| 31 | 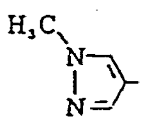 | H | −CH=CH−CH=CH− | =N− | $NO_2$ | |
| 32 | 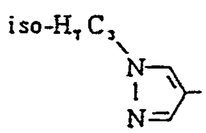 | H | −CH=CH−CH=CH− | =N− | $NO_2$ | |
| 33 | 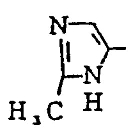 | H | −CH=CH−CH=CH− | =N− | $NO_2$ | |
| 34 | 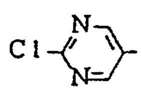 | H | −CH=CH−CH=CH− | =N− | $NO_2$ | |
| 35 | 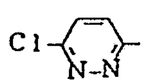 | H | −CH=CH−CH=CH− | =N− | $NO_2$ | |
| 36 | 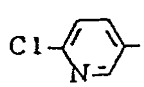 | H | −CH=CH−O− | =N− | $NO_2$ | |
| 37 | 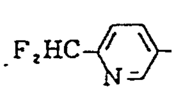 | H | −CH=CH−S− | =N− | $NO_2$ | |
| 38 | 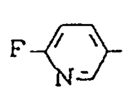 | H | −CH=CH−CH=N− | =N− | $NO_2$ | |

| Verbindung Nr. | W | R | N-Seite T C-Seite | Y | Z |
|---|---|---|---|---|---|
| 39 | 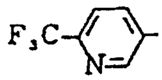 | H | Cl<br>–N=C–CH=CH– | =N– | $NO_2$ |
| 40 | 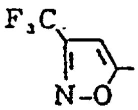 | H | –CH=CH–S– | =N– | $NO_2$ |
| 41 | 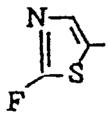 | H | –CH=CH–S– | =N– | $NO_2$ |
| 42 | 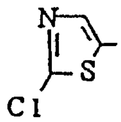 | H | –CH=CH–S– | =N– | $NO_2$ |
| 43 | 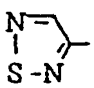 | H | –CH=CH–S– | =N– | $NO_2$ |
| 44 | 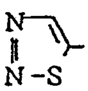 | H | –CH=CH–S– | =N– | $NO_2$ |
| 45 | 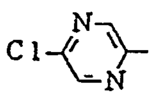 | H | –CH=CH–S– | =N– | $NO_2$ |
| 46 | 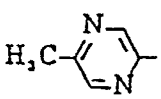 | H | –CH=CH–NH– | =N– | $NO_2$ |
| 47 | 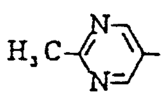 | H | –CH=CH–CH=N– | =N– | $NO_2$ |
| 48 | 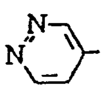 | H | Cl<br>–CH=C–CH=N– | =N– | $NO_2$ |
| 49 | 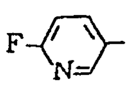 | H | –CH=CH–CH=CH– | =N– | CN |
| 50 | 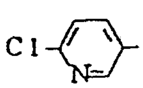 | $CH_3$ | –CH=CH–CH=CH– | =N– | CN |
| 51 | 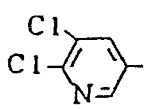 | H | –CH=CH–CH=CH– | =N– | CN |
| 52 | 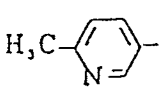 | H | $CH_3$<br>–CH=CH–CH=C– | =N– | CN |

| Verbindung Nr. | W | R | N-Seite T C-Seite | Y | Z |
|---|---|---|---|---|---|
| 53 | 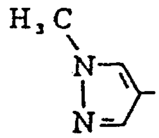 | H | –CH=CH–CH=CH– | =N– | CN |
| 54 | 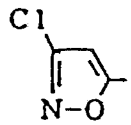 | H | –CH=CH–CH=CH– | =N– | CN |
| 55 | 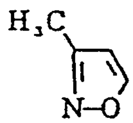 | H | –CH=CH–CH=CH– | =N– | CN |
| 56 | 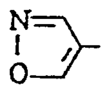 | H | –CH=CH–CH=CH– | =N– | CN |
| 57 | 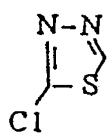 | H | –CH=CH–CH=CH– | =N– | CN |
| 58 | 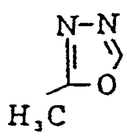 | H | –CH=CH–CH=CH– | =N– | CN |
| 59 | 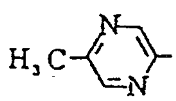 | H | –CH=CH–CH=CH– | =N– | CN |
| 60 | 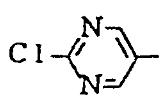 | H | –CH=CH–CH=CH– | =N– | CN |
| 61 | 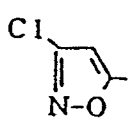 | H | –CH=CH–S– | =N– | CN |
| 62 | 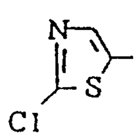 | H | –CH=CH–S– | =N– | CN |
| 63 | 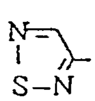 | H | –CH=CH–S– | =N– | CN |

| Verbindung Nr. | W | R | N-Seite T C-Seite | Y | Z |
|---|---|---|---|---|---|
| 64 | 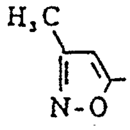 | H | –CH=CH–CH=N– | =N– | CN |
| 65 | 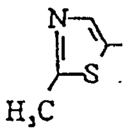 | H | $CH_3$ $CH_3$<br>–CH= CH–C=N– | =N– | CN |
| 66 | 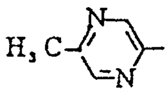 | H | –CH=CH–S– | =N– | CN |
| 67 | 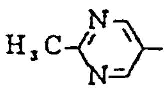 | H | –CH=CH–S– | =N– | CN |
| 68 | 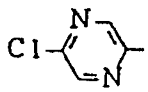 | H | –CH=CH–CH=N– | =N– | CN |
| 69 | 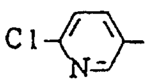 | H | –CH=CH–CH=CH– | =CH– | $NO_2$ |
| 70 | 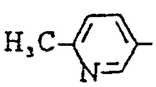 | H | –CH=CH–CH=CH– | =CH– | $NO_2$ |
| 71 | 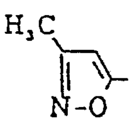 | H | –CH=CH–CH=CH– | =CH– | $NO_2$ |
| 72 | 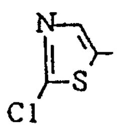 | H | –CH=CH–CH=CH– | =CH– | $NO_2$ |
| 73 | 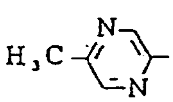 | H | –CH=CH–CH=CH– | =CH– | $NO_2$ |
| 74 | 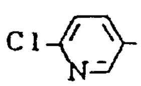 | H | –CH=CH–CH=N– | =CH– | $NO_2$ |
| 75 | 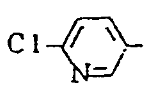 | H | –CH=N–CH=CH– | =CH– | $NO_2$ |
| 76 | 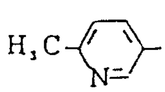 | H | –O–N=CH– | =CH– | $NO_2$ |

| Verbindung Nr. | W | R | N-Seite T C-Seite | Y | Z |
|---|---|---|---|---|---|
| 77 | 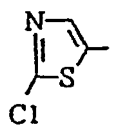 | H | $-CH=N-CH=CH-$ | $=CH-$ | $NO_2$ |
| 78 | 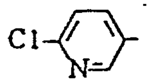 | H | $-CH=CH-S$ | $=CH-$ | $NO_2$ |
| 79 | 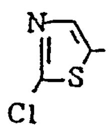 | H | $-CH=CH-S-$ | $=CH-$ | $NO_2$ |
| 80 | 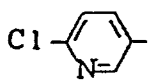 | H | $-CH=CH-NH-$ | $=CH-$ | $NO_2$ |
| 81 | 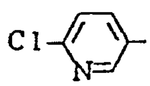 | H | $-CH=CH-CH=CH-$ | $=CH-$ | CN |
| 82 | 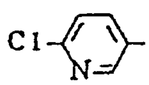 | H | $-CH=CH-CH=N-$ | $=CH-$ | CN |
| 83 | 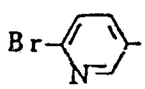 | H | $-CH=CH-CH=N-$ | $=CH-$ | CN |
| 84 | 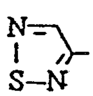 | H | $-CH=CH-CH=N-$ | $=CH-$ | CN |
| 85 | 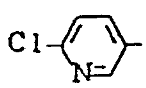 | H | $-CH=CH-NH-$ | $=CN-$ | CN |
| 86 | 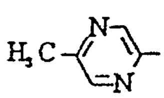 | H | $-CH_2-N=CH-$ | $=CH-$ | CN |
| 87 | 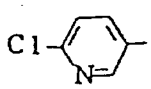 | H | $-CH=CH-S-$ | $=CH-$ | CN |

| Verbindung Nr. | W | R | N-Seite T C-Seite | Y | Z | |
|---|---|---|---|---|---|---|
| 88 | 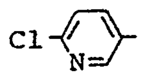 | H | -CH=CH-NH- | =N- | $NO_2$ | Schmp. 186- 189 °C |
| 89 | 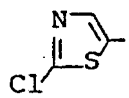 | H | -CH=CH-NH- | =N- | $NO_2$ | Schmp. 156- 157 °C |

Biologische Tests

Vergleichs-Verbindungen

A-1: 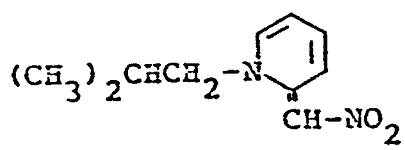

A-2: 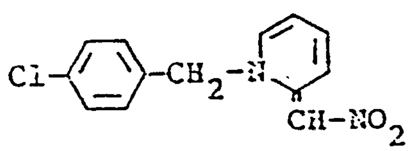

A-3: 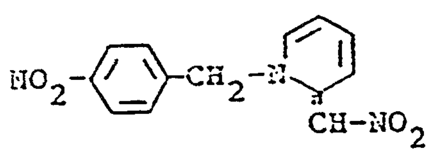

(A-1, A-2 und A-3: die in der JP-OS 29 570/1975 beschriebenen Verbindungen);

B-1: 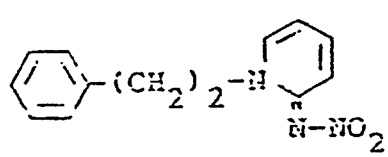

(B-1: die in J. Med. Chem. 1971, Band 14 , Seiten 988-990, beschriebene Verbindung).

Beispiel 3 (Biologischer Test)

Test mit gegen Organophosphor-Mittel resistenten Nephotettix cincticeps:

Herstellung einer Test-Chemikalie :

| | | |
|---|---|---|
| Lösungsmittel: | 3 Gew.-Teile | Xylol |
| Emulgator: | 1 Gew.-Teil | Polyoxyethylen-alkylphenylether |

Zur Herstellung eines geeigneten Test-Präparats der Chemikalie wurde 1 Gew.-Teil des Wirkstoffs mit der oben bezeichneten Menge Lösungsmittel, das die oben angegebene Menge Emulgator enthielt, vermischt. Die Mischung wurde mit Wasser auf eine vorher festgesetzte Konzentration verdünnt.

Test-Verfahren :

Auf Reispflanzen von etwa 10 cm Höhe, die jeweils in Töpfen von 12 cm Durchmesser gezogen worden waren, wurde eine Wasser-Verdünnung des Wirkstoffs mit einer vorher festgelegten Konzentration, die wie oben beschrieben hergestellt worden war, in einer Menge von 10 ml pro Topf gesprüht. Die aufgesprühte Chemikalie wurde trocknen gelassen, und über jeden der Töpfe wurde ein Drahtkorb von 7 cm Durchmesser und 14 cm Höhe gestülpt, und 30 ausgewachsene weibliche Exemplare von Nephotettix cincticeps, die gegen Organophosphor-Mitteln Resistenz zeigten, wurden unter dem Drahtkorb ausgesetzt. Die Töpfe wurden jeweils in einem Raum mit konstanter Temperatur aufbewahrt, die Zahl der toten Insekten wurde 24 Stunden später bestimmt, und das Tötungsverhältnis wurde berechnet.

In diesem Test zeigten die Verbindungen Nr. 1, 2, 8, 10, 20, 23, 25, 26 ein Tötungsverhältnis von 100 % bei einer Wirkstoff-Konzentration von 8 ppm, wohingegen die Vergleichsverbindungen A-1, A-3 und B-1 bei 40 ppm keine Wirkung zeigten und die Vergleichsverbindung A-2 bei einer Konzentration von 8 ppm keine Wirkung zeigte.

Beispiel 4 (Biologischer Test)

Test mit Laternenträgern

Test-Verfahren :

Eine Wasser-Verdünnung jeder der Wirkstoffe mit einer vorher festgelegten Konzentration, die wie in Beispiel 3 beschrieben hergestellt worden war, wurde auf Reispflanzen von etwa 10 cm Höhe, die jeweils in Töpfen von 12 cm Durchmesser gezogen worden waren, in einer Menge von 10 ml pro Topf gesprüht. Nach dem Trocknen der aufgesprühten Chemikalie wurde über jeden der Töpfe ein Drahtkorb von 7 cm Durchmesser und 14 cm Höhe gestülpt, und 30 ausgewachsene weibliche Exemplare von Nilaparvata lugens wurden unter dem Korb ausgesetzt. Die Töpfe wurden in einem Inkubator aufbewahrt, und die Zahl der toten Insekten wurde zwei Tage später bestimmt. Das Tötungsverhältnis wurde berechnet.

In der gleichen Weise wurde das Tötungsverhältnis für Sogatella furcifera und organophosphor-resistente Laodelphax striatellus berechnet.

In diesem Test zeigten beispielsweise die Verbindungen Nr. 1, 2, 8 und 10 gemäß der vorliegenden Patentanmeldung ein Tötungsverhältnis von 100 % gegenüber N. lugens, L. striatellus und S. furcifera bei einer Wirkstoff-Konzentration von 40 ppm, wohingegen die Verbindungen der Vergleichsbeispiele A-1, A-2, A-3 und B-1 bei den oben genannten Test-Systemen entweder unwirksam oder beträchtlich weniger wirksam waren.

Beispiel 5 (Biologischer Test)

Test mit Myzus persicae (grünen Pfirsichblattläusen), die gegen Organophosphor-Mittel und Carbamat-Mittel Resistenz zeigten:

Test-Verfahren :

Gezüchtete grüne Pfirsichblattläuse wurden auf Auberginen-Setzlingen (schwarze längliche Auberginen) von etwa 20 cm Höhe ausgesetzt, die in unglasierten Töpfen von 15 cm Durchmesser gezogen worden waren (etwa 200 Blattläuse pro Setzling). Einen Tag nach dem Aussetzen wurde eine Wasser-Verdünnung jeder der Wirkstoffe mit einer vorher festgelegten Konzentration, die wie in Beispiel 3 beschrieben hergestellt worden war, in genügender Menge mit Hilfe einer Spritzpistole auf die Pflanzen aufgesprüht. Nach dem Sprühen wurden die Töpfe in einem Gewächshaus bei 28 °C stehen gelassen. 24 h nach dem Sprühen wurde das Tötungsverhältnis berechnet. Für jede Verbindung wurde der vorstehende Test mit zwei Wiederholungen durchgeführt.

In diesem Test zeigten beispielsweise die Verbindungen Nr. 1, 2, 8 und 10 gemäß der vorliegenden Anmeldung ein Tötungsverhältnis von 100 % bei einer Konzentration von 200 ppm des Wirkstoffs, wohingegen die Verbindungen der Vergleichsbeispiele A-1, A-2, A-3 und B-1 gegen Myzus persicae entweder unwirksam oder beträchtlich weniger wirksam waren.

## Ansprüche

1. Heterocyclische Verbindungen der Formel (I)

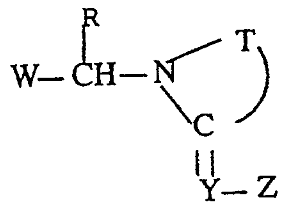

(I)

in welcher

W eine Pyridyl-Gruppe mit wenigstens einem Substituenten ausgewählt aus Halogen-Atomen, $C_1$-$C_4$-Alkyl-Gruppen, die gegebenenfalls durch Halogen substituiert sind, $C_1$-$C_4$-Alkoxy-Gruppen, die gegebenenfalls durch Halogen substituiert sind, $C_2$-$C_4$-Alkenyl-Gruppen, die gegebenenfalls durch Halogen substituiert sind, $C_1$-$C_4$-Alkylsulfinyl-Gruppen, $C_1$-$C_4$-Alkylsulfonyl-Gruppen und $C_3$-$C_4$-Alkinyl-Gruppen oder eine heterocyclische Gruppe, die zwei Hetero-Atome ausgewählt aus Sauerstoff-, Schwefelund Stickstoff-Atomen enthält, wobei wenigstens eines der -Hetero-Atome ein Stickstoff-Atom ist, und die gegebenenfalls durch einen Substituenten ausgewählt aus Halogen-Atomen, $C_1$-$C_4$-Alkyl-Gruppen, die gegebenenfalls durch Halogen substituiert sind, $C_1$-$C_4$-Alkoxy-Gruppen, die gegebenenfalls durch Halogen substituiert sind, $C_2$-$C_4$-Alkenyl-Gruppen, die gegebenenfalls durch Halogen substituiert sind, $C_1$-$C_4$-Alkylsulfinyl-Gruppen, $C_1$-$C_4$-Alkylsulfonyl-Gruppen und $C_3$-$C_4$-Alkinyl-Gruppen substituiert sein kann, ist
R ein Wasserstoff-Atom oder eine Methyl-Gruppe bezeichnet,
Y =N- bezeichnet,
Z eine Nitro-Gruppe oder eine Cyano-Gruppe bezeichnet und
T 3 oder 4 Ring-Glieder eines 5- oder 6-gliedrigen ungesättigten heterocyclischen Ringes bezeichnet, den diese zusammen mit dem benachbarten Kohlenstoff-Atom und dem benachbarten Stickstoff-Atom bilden, wobei der 5- oder 6-gliedrige ungesättigte heterocyclische Ring 1 bis 2 Hetero-Atome enthält, die aus Schwefel- und Stickstoff-Atomen ausgewählt sind und von denen wenigstens eines ein Stickstoff-Atom ist, wobei die 3 oder 4 Ring-Glieder gegebenenfalls durch wenigstens ein Halogen-Atom bzw. eine gegebenenfalls durch Halogen substituierte $C_1$-$C_4$-Alkyl-Gruppe substituiert sind.

2. Verbindungen nach Anspruch 1, dadurch gekennzeichnet, daß

W eine Pyridyl-Gruppe mit einem Substituenten ausgewählt aus Fluoro, Chloro, Bromo, Methyl, Ethyl, Trifluoromethyl, Methoxy, Trifluoromethoxy, Vinyl, Allyl, Methylsulfinyl, Methylsulfonyl und Propargyl oder

eine ein Sauerstoff- oder Schwefel-Atom und ein Stickstoff-Atom enthaltende, 5-gliedrige heterocyclische Gruppe, die gegebenenfalls substituiert ist durch einen Substituenten ausgewählt aus Fluoro, Chloro, Bromo, Methyl, Ethyl, Trifluoromethyl, Methoxy, Trifluoromethoxy, Vinyl, Allyl, Methylsulfinyl, Methylsulfonyl und Propargyl, ist,

R ein Wasserstoff-Atom oder eine Methyl-Gruppe bezeichnet,

Y für =N- steht,

Z eine Nitro-Gruppe oder eine cyano-Gruppe bezeichnet und

T 3 oder 4 Ring-Glieder eines Imidazolin-, Thiazol-in-, Dihydropyridin- oder Dihydropyrimidin-Rings bezeichnen, den diese zusammen mit dem benachbarten Kohlenstoff-Atom und dem benachbarten Stickstoff-Atom bilden, wobei die Ring-Glieder gegebenenfalls durch Chloro oder Methyl substituiert sind.

**3.** Heterocyclische Verbindung nach Ansprüchen 1 bis 3, ausgewählt aus der Gruppe der nachstehenden Verbindungen A) bis G)

1-(2-Chloro-5-pyridylmethyl)-2-nitroimino-1,2-dihydropyridin der nachstehenden Formel

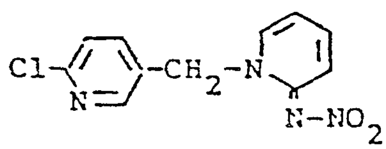

A)

,

1-(2-Chloro-5-pyridylmethyl)-5-methyl-2-nitroimino-1,2-dihydro-pyridin der nachstehenden Formel

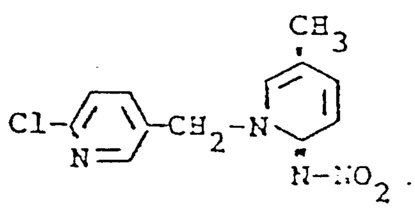

B)

,

1-(3-Methyl-5-isoxazolylmethyl)-2-nitroimino-1,2-dihydro-pyridin der nachstehenden Formel

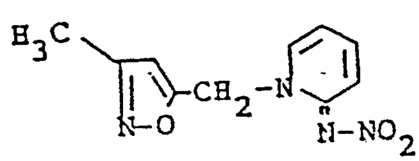

C)

,

3-(2-Chloro-5-pyridylmethyl)-2-nitroimino-4-thiazolin der nachstehenden Formel

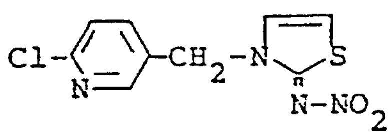

D)

,

1-(2-Chloro-5-pyridylmethyl)-2-cyanoimino-1,2-dihydro-pyridin der nachstehenden Formel

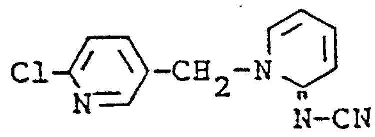

E) ,

1-(2-Chloro-5-pyridylmethyl)-2-cyanoimino-1,2-dihydropyrimidin der nachstehenden Formel

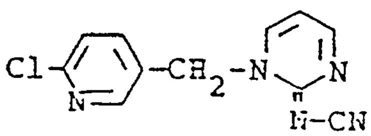

F) ,

1-(2-Chloro-5-thiazolylmethyl)-2-cyanoimino-1,2-dihydro-pyridin der nachstehenden Formel

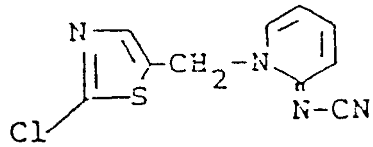

G) .

4. Verfahren zur Herstellung von heterocyclischen Verbindungen der Formel (I)

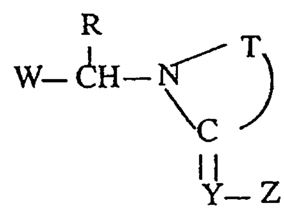

(I)

in welcher

W eine Pyridyl-Gruppe mit wenigstens einem Substituenten ausgewählt aus Halogen-Atomen, $C_1$-$C_4$-Alkyl-Gruppen, die gegebenenfalls durch Halogen substituiert sind, $C_1$-$C_4$-Alkoxy-Gruppen, die gegebenenfalls durch Halogen substituiert sind, $C_2$-$C_4$-Alkenyl-Gruppen, die gegebenenfalls durch Halogen substituiert sind, $C_1$-$C_4$-Alkylsulfinyl-Gruppen, $C_1$-$C_4$-Alkylsulfonyl-Gruppen und $C_3$-$C_4$-Alkinyl-Gruppen oder eine heterocyclische Gruppe, die zwei Hetero-Atome ausgewählt aus Sauerstoff-, Schwefel-und Stickstoff-Atomen enthält, wobei wenigstens eines der Hetero-Atome ein Stickstoff-Atom ist, und die gegebenenfalls durch einen Substituenten ausgewählt aus Halogen-Atomen, $C_1$-$C_4$-Alkyl-Gruppen, die gegebenenfalls durch Halogen substituiert sind, $C_1$-$C_4$-Alkoxy-Gruppen, die gegebenenfalls durch Halogen substituiert sind, $C_2$-$C_4$-Alkenyl-Gruppen, die gegebenenfalls durch Halogen substituiert sind, $C_1$-$C_4$-Alkylsulfinyl-Gruppen, $C_1$-$C_4$-Alkylsulfonyl-Gruppen und $C_3$-$C_4$-Alkinyl-Gruppen substituiert sein kann, ist

R ein Wasserstoff-Atom oder eine Methyl-Gruppe bezeichnet,

Y =N- bezeichnet,

Z eine Nitro-Gruppe oder eine Cyano-Gruppe bezeichnet und

T 3 oder 4 Ring-Glieder eines 5- oder 6-gliedrigen ungesättigten heterocyclischen Ringes bezeichnet, den diese zusammen mit dem benachbarten Kohlenstoff-Atom und dem benachbarten Stickstoff-Atom bilden, wobei der 5- oder 6-gliedrige ungesättigte heterocyclische Ring 1 bis 2 Hetero-Atome enthält, die aus Schwefel- und Stickstoff-Atomen ausgewählt sind und von denen wenigstens, eines ein Stickstoff-Atom ist, wobei die 3 oder 4 Ring-Glieder gegebenenfalls durch wenigstens ein Halogen-Atom bzw. eine gegebenenfalls durch Halogen substituierte $C_1$-$C_4$-Alkyl-

Gruppe substituiert sind, dadurch gekennzeichnet, daß
a) Verbindungen der Formel (II)

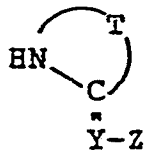

(II)

in der Y, Z und T die angegebenen Bedeutungen haben, mit Verbindungen der Formel (III)

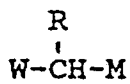

(III),

in der R und W die oben angegebenen Bedeutungen haben und M fur ein Halogen-Atom oder die Gruppe $-OSO_2-R'$ steht, in der R' eine Alkyl- oder ArylGruppe bezeichnet,
in Gegenwart eines inerten Lösungsmittels und gegebenenfalls in Gegenwart einer Base umgesetzt werden.

5. Insektizide Mittel, dadurch gekennzeichnet, daß sie wenigstens eine heterocyclische Verbindung der Formel (I) enthalten.

6. Verfahren zur Bekämpfung schädlicher Insekten, dadurch gekennzeichnet, daß man heterocyclische Verbindungen der Formel (I) auf die schädlichen Insekten und/oder deren Lebensraum einwirken läßt.

7. Verwendung neuer heterocyclischer Verbindungen der Formel (I) zur Bekämpfung schädlicher Insekten.

8. Verfahren zur Herstellung insektizider Mittel, dadurch gekennzeichnet, daß neue heterocyclische Verbindungen der Formel (I) mit Streckmitteln und/oder grenzflächenaktiven Mitteln vermischt werden.

## Claims

1. Heterocyclic compounds of the formula (I)

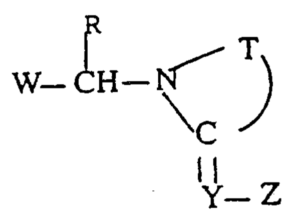

(I)

in which
W is a pyridyl group having at least one substituent selected from amongst halogen atoms, $C_1$-$C_4$-alkyl groups which are optionally substituted by halogen, $C_1$-$C_4$-alkoxy groups which are optionally substituted by halogen, $C_2$-$C_4$-alkenyl groups which are optionally substituted by halogen, $C_1$-$C_4$-alkylsulphinyl groups, $C_1$-$C_4$-alkylsulphonyl groups and $C_3$-$C_4$-alkinyl groups or a heterocyclic group which contains two hetero atoms selected from amongst oxygen, sulphur and nitrogen atoms, at least one of the hetero atoms being a nitrogen atom, and which can optionally be substituted by a substituent selected from amongst halogen atoms, $C_1$-$C_4$-alkyl groups which are optionally substituted by halogen, $C_1$-$C_4$-alkoxy groups which are optionally substituted by halogen, $C_2$-$C_4$-alkenyl groups which are optionally substituted by halogen, $C_1$-$C_4$-alkylsulphinyl groups, $C_1$-$C_4$-alkylsulphonyl groups and $C_3$-$C_4$-alkinyl groups,

R denotes a hydrogen atom or a methyl group,
Y denotes =N-,
Z denotes a nitro group or a cyano group and
T denotes 3 or 4 ring members of a 5- or 6-membered unsaturated heterocyclic ring which is formed by these ring members together with the adjacent carbon atom and the adjacent nitrogen atom, the 5- or 6-membered unsaturated heterocyclic ring containing 1 to 2 hetero atoms which are selected from amongst sulphur and nitrogen atoms and of which at least one is a nitrogen atom, the 3 or 4 ring members optionally being substituted by at least one halogen atom or an optionally halogen-substituted $C_1$-$C_4$-alkyl group.

2. Compounds according to Claim 1, characterised in that
W is a pyridyl group with a substituent selected from amongst fluoro, chloro, bromo, methyl, ethyl, trifluoromethyl, methoxy, trifluoromethoxy, vinyl, allyl, methylsulphinyl, methylsulphonyl and propargyl, or
a 5-membered heterocyclic group which contains an oxygen or sulphur atom and a nitrogen atom and which is optionally substituted by a substituent selected from amongst fluoro, chloro, bromo, methyl, ethyl, trifluoromethyl, methoxy, trifluoromethoxy, vinyl, allyl, methylsulphinyl, methylsulphonyl and propargyl,
R denotes a hydrogen atom or a methyl group,
Y represents =N-,
Z denotes a nitro group or a cyano group and
T denote 3 or 4 ring members of an imidazoline, thiazoline, dihydropyridine or dihydropyrimidine ring which is formed by these ring members together with the adjacent carbon atom and the adjacent nitrogen atom, the ring members optionally being substituted by chloro or methyl.

3. Heterocyclic compound according to Claims 1 to 3, selected from amongst the group of the compounds A) to G) below
1-(2-chloro-5-pyridylmethyl)-2-nitroimino-1,2-dihydropyridine, of the formula below

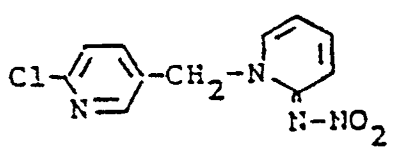

A)

1-(2-chloro-5-pyridylmethyl)-5-methyl-2-nitroimino-1,2-dihydro-pyridine, of the formula below

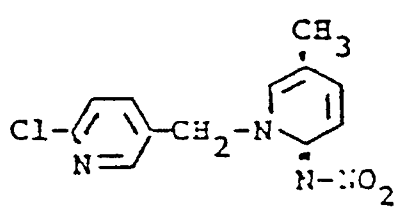

B)

1-(3-methyl-5-isoaxazolylmethyl)-2-nitroimino-1,2-dihydro-pyridine, of the formula below

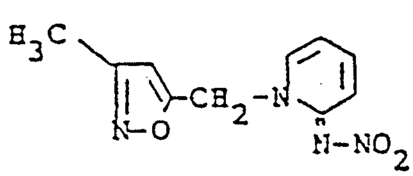

C)

3-(2-chloro-5-pyridylmethyl)-2-nitroimino-4-thiazoline, of the formula below

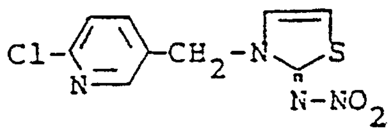

D)

1-(2-chloro-5-pyridylmethyl)-2-cyanoimino-1,2-dihydro-pyridine, of the formula below

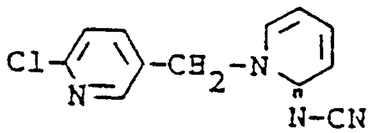

E)

1-(2-chloro-5-pyridylmethyl)-2-cyanoimino-1,2-dihydro-pyrimidine, of the formula below

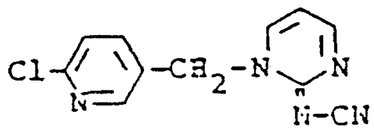

F)

1-(2-chloro-5-thiazolylmethyl)-2-cyanoimino-1,2-dihydro-pyridine, of the formula below

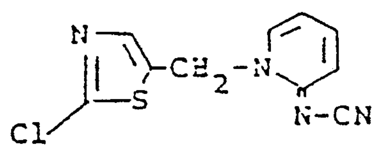

G)

4. Process for the preparation of heterocyclic compounds of the formula (I)

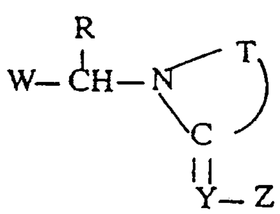

(I)

in which

W is a pyridyl group having at least one substituent selected from amongst halogen atoms, $C_1$-$C_4$-alkyl groups which are optionally substituted by halogen, $C_1$-$C_4$-alkoxy groups which are optionally substituted by halogen, $C_2$-$C_4$-alkenyl groups which are optionally substituted by halogen, $C_1$-$C_4$-alkylsulphinyl groups, $C_1$-$C_4$-alkylsulphonyl groups and $C_3$-$C_4$-alkinyl groups or a heterocyclic group which contains two hetero atoms selected from amongst oxygen, sulphur and nitrogen atoms, at least one of the hetero atoms being a nitrogen atom, and which can optionally be substituted by a substituent selected from amongst halogen atoms, $C_1$-$C_4$-alkyl groups which are optionally substituted by halogen, $C_1$-$C_4$-alkoxy groups which are optionally substituted by halogen, $C_2$-$C_4$-alkenyl groups which are optionally substituted by halogen, $C_1$-$C_4$,-alkylsulphinyl groups, $C_1$-$C_4$-alkylsulphonyl groups and $C_3$-$C_4$-alkinyl groups,

R denotes a hydrogen atom or a methyl group,

Y denotes =N-,

Z denotes a nitro group or a cyano group and

T denotes 3 or 4 ring members of a 5- or 6-membered unsaturated heterocyclic ring which is

formed by these ring members together with the adjacent carbon atom and the adjacent nitrogen atom, the 5- or 6-membered unsaturated heterocyclic ring containing 1 to 2 hetero atoms which are selected from amongst sulphur and nitrogen atoms and of which at least one is a nitrogen atom, the 3 or 4 ring members optionally being substituted by at least one halogen atom or an optionally halogen-substituted $C_1$-$C_4$-alkyl group,
characterised in that
a) compounds of the formula (II)

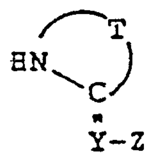

(II)

in which Y, Z and T have the above-mentioned meanings, are reacted with compounds of the formula (III)

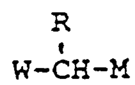

(III)

in which R and W have the above-mentioned meanings and M represents a halogen atom or the group $-OSO_2-R'$ in which R' denotes an alkyl or aryl group, in the presence of an inert solvent, and, if appropriate, in the presence of a base.

5. Insecticidal agents, characterised in that they contain at least one heterocyclic compound of the formula (I).

6. Method of controlling harmful insects, characterised in that heterocyclic compounds of the formula (I) are allowed to act on the harmful insects and/or their environment.

7. Use of novel heterocyclic compounds of the formula (I) for controlling harmful insects.

8. Process for the preparation of insecticidal agents, characterised in that novel heterocyclic compounds of the formula (1) are mixed with extenders and/or surface-active agents.

## Revendications

1. Composés hétérocycliques de formule (I)

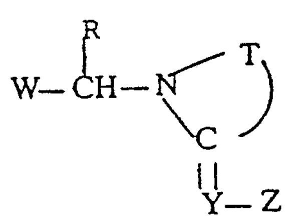

(I)

dans laquelle
W est un groupe pyridyle portant au moins un substituant choisi entre des atomes d'halogènes, des groupes alkyle en $C_1$ à $C_4$ qui sont substitués le cas échéant par un halogène, des groupes alkoxy en $C_1$ à $C_4$ qui sont substitués le cas échéant par un halogène, des groupes alcényle en $C_2$ à $C_4$ qui sont substitués le cas échéant par un halogène, des groupes alkylsulfinyle en $C_1$ à $C_4$, des groupes alkylsulfonyle en $C_1$ à $C_4$ et des groupes alcynyle en $C_3$ ou $C_4$ ou un groupe hétérocycli-

que qui contient deux hétéro-atomes choisis entre des atomes d'oxygène, de soufre et d'azote, l'un au moins des hétéro-atomes étant un atome d'azote, et qui peut être substitué le cas échéant par un substituant choisi entre des atomes d'halogènes, des groupes alkyle en $C_1$ à $C_4$ qui sont éventuellement substitués par un halogène, des groupes alkoxy en $C_1$ à $C_4$ qui sont éventuellement substitués par un halogene, des groupes alcényle en $C_2$ à $C_4$ qui sont éventuellement substitués par un halogène, des groupes alkylsulfinyle en $C_1$ à $C_4$, des groupes alkylsulfonyle en $C_1$ à $C_4$ et des groupes alcynyle en $C_3$ ou $C_4$,
R est un atome d'hydrogène ou un groupe méthyle,
Y représente =N-,
Z est un groupe nitro ou un groupe cyano et
T représente 3 ou 4 chaînons d'un noyau hétérocyclique non saturé pentagonal ou hexagonal que forment ces chaînons conjointement avec l'atome contigu de carbone et l'atome contigu d'azote, le noyau hétérocyclique non saturé pentagonal ou hexagonal contenant 1 ou 2 hétéro-atomes qui sont choisis entre des atomes de soufre et d'azote et dont l'un au moins est un atome d'azote, les 3 ou 4 chaînons étant substitués le cas échéant par au moins un atome d'halogène ou un groupe alkyle en $C_1$ à $C_4$ éventuellement substitué par un halogène.

2. Composés suivant la revendication 1, caractérisés en ce que
W est un groupe pyridyle portant un substituant choisi entre des substituants fluoro, chloro, bromo, méthyle, éthyle, trifluorométhyle, méthoxy, trifluorométhoxy, vinyle, allyle, méthylsulfinyle, méthylsulfonyle et propargyle ou
un groupe hétérocyclique pentagonal contenant un atome d'oxygène ou de soufre et un atome d'azote, qui porte éventuellement un substituant choisi entre des substituants fluoro, chloro, bromo, méthyle, éthyle, trifluorométhyle, méthoxy, trifluorométhoxy, vinyle, allyle, méthylsulfinyle, méthylsulfonyle et propargyle,
R est un atome d'hydrogène ou un groupe méthyle,
Y représente =N-,
Z est un groupe nitro ou un groupe cyano et
T représente 3 ou 4 chaînons d'un noyau d'imidazoline, de thiazoline, de dihydropyridine ou de dihydropyrimidine, que forment ces chaînons conjointement avec l'atome contigu de carbone ou l'atome contigu d'azote, les chaînons du noyau étant éventuellement substitués par un radical chloro ou méthyle.

3. Composé hétérocyclique suivant les revendications 1 à 3, choisi dans le groupe des composés A) à G) suivants
1-(2-chloro-5-pyridylméthyl)-2-nitro-imino-1,2-dihydropyridine de formule suivante

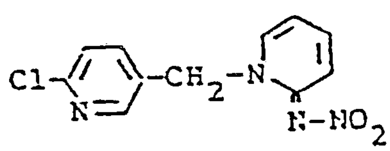

A) ,

1-(2-chloro-5-pyridylméthyl)-5-méthyl-2-nitroimino-1,2-dihydropyridine de formule suivante

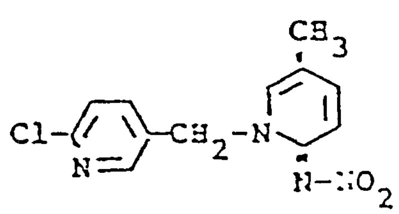

B) ,

1-(3-méthyl-5-isoxazolylméthyl)-2-nitro-imino-1,2-dihydropyridine de formule suivante

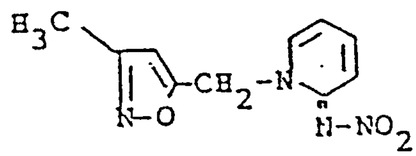

C)

,

3-(2-chloro-5-pyridylméthyl)-2-nitro-imino-4-thiazoline de formule suivante

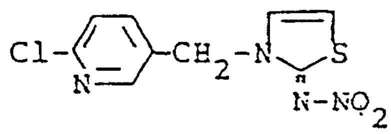

D)

,

1-(2-chloro-5-pyridylméthyl)-2-cyano-imino-1,2-dihydropyridine de formule suivante

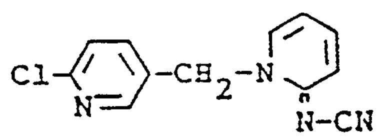

E)

,

1-(2-chloro-5-pyridylméthyl)-2-cyano-imino-1,2-dihydropyrimidine de formule suivante

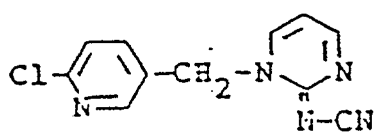

F)

,

1-(2-chloro-5-thiazolylméthyl)-2-cyano-imino-1,2-dihydropyridine de formule suivante

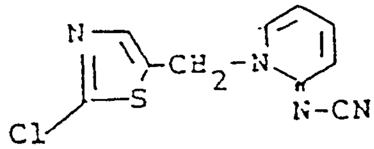

G)

**4.** Procédé de production de composés hétérocycliques de formule (I)

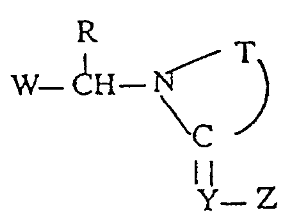

(I)

dans laquelle

W est un groupe pyridyle portant au moins un substituant choisi entre des atomes d'halogènes, des groupes alkyle en $C_1$ à $C_4$ qui sont substitués le cas échéant par un halogène, des groupes alkoxy

en $C_1$ à $C_4$ qui sont substitués le cas échéant par un halogène, des groupes alcényle en $C_2$ à $C_4$ qui sont substitués le cas échéant par un halogène, des groupes alkylsulfinyle en $C_1$ à $C_4$, des groupes alkylsulfonyle en $C_1$ à $C_4$ et des groupes alcynyle en $C_3$ ou $C_4$ ou un groupe hétérocyclique qui contient deux hétéro-atomes choisis entre des atomes d'oxygène, de soufre et d'azote, l'un au moins des hétéro-atomes étant un atome d'azote, et qui peut être substitué le cas échéant par un substituant choisi entre des atomes d'halogènes, des groupes alkyle en $C_1$ à $C_4$ qui sont éventuellement substitués par un halogène, des groupes alkoxy en $C_1$ à $C_4$ qui sont éventuellement substitués par un halogène, des groupes alcényle en $C_2$ à $C_4$ qui sont éventuellement substitués par un halogène, des groupes alkylsulfinyle en $C_1$ à $C_4$, des groupes alkylsulfonyle en $C_1$ à $C_4$ et des groupes alcynyle en $C_3$ ou $C_4$,

R est un atome d'hydrogene ou un groupe méthyle,

Y représente =N-,

Z est un groupe nitro ou un groupe cyano et

T représente 3 ou 4 chaînons d'un noyau hétérocyclique non saturé pentagonal ou hexagonal que forment ces chaînons conjointement avec l'atome contigu de carbone et l'atome contigu d'azote, le noyau hétérocyclique non saturé pentagonal ou hexagonal contenant 1 ou 2 hétéro-atomes qui sont choisis entre des atomes de soufre et d'azote et dont l'un au moins est un atome d'azote, les 3 ou 4 chaînons étant substitués le cas échéant par au moins un atome d'halogène ou un groupe alkyle en $C_1$ à $C_4$ éventuellement substitué par un halogène,

caractérisé en ce que

a) on fait réagir des composés de formule (II)

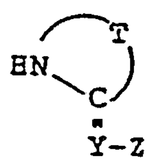

(II) ,

dans laquelle Y, Z et T ont les définitions indiquées, avec des composés de formule (III)

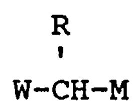

(III),

dans laquelle R et W ont les définitions indiquées ci-dessus et M est un atome d'halogène ou le groupe $-OSO_2-R'$, où R' désigne un groupe alkyle ou aryle, en présence d'un solvant inerte et, le cas échéant, en présence d'une base.

**5.** Compositions insecticides, caractérisées en ce qu'elles contiennent au moins un composé hétérocyclique 4de formule (I).

**6.** Procédé pour combattre des insectes parasites, caractérisé en ce qu'on fait agir des composés hétérocycliques de formule (I) sur les insectes parasites et/ou sur leur milieu.

**7.** Utilisation de composés hétérocycliques nouveaux de formule (I) pour combattre des insectes parasites.

**8.** Procédé de préparation de compositions insecticides, caractérisé en ce qu'on mélange des composés hétérocycliques nouveaux de formule (I) avec des diluants et/ou des agents tensio-actifs.